# EUROPEAN PATENT APPLICATION

(11) **EP 4 578 456 A1**
(43) Date of publication of application: **02.07.2025**
(21) Application number: 23220796.9
(22) Date of filing: 29.12.2023
(51) Int. Cl.: A61K 36/53, A61K 9/50, A61P 9/10

(54) **MELISSA OFFICINALIS PLANT-DERIVED NANOVESICLE COMPOSITIONS AND USES THEREOF**

(71) Applicant: Lietuvos sveikatos mokslu universitetas, 44307 Kaunas (LT)
(72) Inventor: Balion, Zbigniev, Kaunas (LT); Zukauskaite, Ineta, Kaunas (LT); Keturakis, Vytenis, Kaunas (LT); Jekabsone, Aiste, Kaunas (LT); Benetis, Rimantas, Kaunas (LT)
(74) Representative: AAA Law

(57) **Abstract**

The present invention relates to nanovesicles isolated from *Melissa officinalis* plant, the compositions comprising said nanovesicles and use of such nanovesicles and compositions. In particular, the nanovesicles for use in therapy and/or medical treatment are provided, preferably for use in prevention and/or treatment of ischaemia/reperfusion damage of cells or tissue. The nanovesicles, as provided herein, are able to enhance cell viability and regulate cell (for example, cardiomyocyte cell) contractility, thus protecting cells and tissues from damage or under stress conditions.

## Description

### FIELD OF INVENTION

The present invention relates to nanovesicles isolated from *Melissa officinalis* plant, and, in particular, use of such nanovesicles in therapy and/or medical treatment, for example, to prevent and/or treat ischemic/reperfusion injuries.

### BACKGROUND OF INVENTION

Ischaemia/reperfusion (I/R) injury of the cardiomyocyte and subsequent mitochondrial damage is closely related to acute coronary syndromes [1]. Myocardium reperfusion by either primary coronary artery angioplasty or coronary artery bypass grafting has become the golden standard in patients with myocardial infarction, which significantly increases the chances of survival and preservation of heart function. However, restored blood flow into an ischaemic area is always accompanied by myocardial injury [2].

Reperfusion injuries can be classified into two groups: reversible (sublethal) injuries, like reperfusion-induced arrhythmias and myocardial stunning (prolonged but reversible contractile dysfunction), and irreversible (lethal) ones, caused by the accelerated necrosis in the tissue that was irreversibly injured by ischaemia (the "oxygen paradox"), microvascular obstruction (no-reflow phenomenon) and lethal reperfusion injury causing the death of cardiomyocytes which were viable at the end of ischaemic events [3].

At the cellular level, both the main targets and propagators of I/R-induced cardiomyocyte injury are mitochondria. Ischaemia primes, and reperfusion further propagates mitochondrial permeability transition pore, resulting in mitochondrial (and, in its turn, cardiomyocyte) functional failure and apoptotic death that can turn into necrosis due to the lack of ATP. Increased necrosis is associated with irreversible myocardial injury and risk of lethal consequences. Therefore, mitochondrial function preservation is key to myocardial recovery after I/R [4].

A specific case of induced I/R injury is cardiac surgery, the outcome of which strongly depends on the efficiency of tissue recovery. Besides, due to the ageing of the population, there is an increasing number of frail cardiac surgery patients with a diminished balance of mitochondrial potency because mitochondrial performance declines with ageing. Surgeries on these patients are more risky because of their compromised cardiac tissue and concomitant diseases. There is a lack of viable and evidence-based clinical practice capable of decreasing mitochondrial injury in cardiac surgery patients and alleviating I/R injury in acute coronary disease and during cardiac surgery operations. This lack of efficient strategies leads to negative postoperative results and a higher incidence of postoperative heart insufficiency.

Lately, many studies have focused on the use of extracellular vesicles as therapeutics. Secreted extracellular vesicles, mostly exosomes, have become promising biological and precision medicine candidates. Exosomes extracted from stem and progenitor cell lines have demonstrated therapeutic efficacy in preclinical models of heart disease. Much research has been put into using exosomes derived from human or animal cells; however, human and animal cell-derived materials pose ethical issues, and their production cost is high. In contrast, plant-derived nanovesicles are animal-free and cheaper; however, little work has been done to investigate their therapeutic potential. Plant-derived nanovesicles carry encapsulated proteins, mRNAs, and secondary metabolites that can interact with human cells and are protected from degradation by lipid bilayer membranes. Besides, the membranous protein-decorated envelope is opsonised by host proteins in the organism and is not immunogenic even when applied interspecies [5]. Such nanovesicles can penetrate deep into tissues and cross biological barriers, carrying their active substances to the cells far from the spot of their application. The above-listed properties make isolated plant-derived nanovesicles a novel plant material with unique efficiency. *Melissa officinalis* (lemon balm) extract is shown to prevent ischemia-induced arrhythmia and reduce cardiac tissue damage size [6]. The bioactive components of this extract are secondary metabolites, mainly phenolic compounds, most of which degrade fast in such aqueous solutions, and their ability to penetrate tissues and cells is relatively low. Therefore, the efficiency of such preparation is not lasting, and concentrations in the heart are not enough for the desired effect.

### SUMMARY OF INVENTION

The present invention relates to nanovesicles isolated from *Melissa officinalis* plant, the compositions comprising said nanovesicles and use of such nanovesicles and compositions. In particular, the nanovesicles for use in therapy and/or medical treatment are provided, preferably for use in prevention and/or treatment of ischaemia/reperfusion damage of cells or tissue. The nanovesicles, as provided herein, are able to enhance cell viability and regulate cell (for example, cardiomyocyte cell) contractility, thus protecting cells and tissues from damage or under stress conditions. In *Melissa officinalis-*derived nanovesicles, secondary metabolites and other biomolecules are protected from environmental damage and can ensure delivery of their bioactive content to cardiac cells.

In a first aspect of the present invention, nanovesicles obtained from *Melissa officinalis* plant are provided.

In a second aspect of the present invention, the nanovesicles from *Melissa officinalis* plant for use in therapy and/or medical treatment are provided. In some embodiments, the nanovesicles are for use in prevention and/or treatment of ischaemia/reperfusion damage of cells or tissue.

In a further aspect of the present invention, a composition for use in prevention and/or treatment of ischaemia/reperfusion damage of cells or tissue, characterised in that the composition contains nanovesicles obtained from *Melissa officinalis* is provided. In some embodiments, the composition further comprises one or more additional active ingredients, provided that said one or more additional active ingredients are not encapsulated in said nanovesicles. In some embodiments, the composition is a cardioplegic composition.

In some embodiments, a pharmaceutical composition containing the nanovesicles obtained from *Melissa officinalis* and a pharmaceutically acceptable excipient is provided.

In a further aspect of the present invention, the use of nanovesicles obtained from *Melissa officinalis* to enhance cardiomyocyte viability and/or regulate cardiomyocyte contractility is provided.

### BRIEF DESCRIPTION OF DRAWINGS

Figure 1. *Melissa officinalis* nanovesicle visualisation using Transmission electron microscopy.
Figure 2. Particle size distribution determined by NTA analysis.
Figure 3. Particle size determined by dynamic light scattering analysis.
Figure 4. Cardiomyocyte: brightfield microscope image on the left and fluorescence on the right - the actin cytoskeleton of a cell stained with phalloidin conjugated with the red fluorescent label TRITC.
Figure 5. Metabolic activity of primary cardiomyocyte culture with *Melissa officinalis* nanovesicles. * - denotes statistically significant difference compared to control when p < 0.05, n=3.
Figure 6. Metabolic activity of cardiac cell culture exposed to hypoxialreoxygenation and *Melissa officinalis* nanovesicles. * - statistical significance compared to normoxia control at p < 0.05, ** denotes statistical significance compared to hypoxia and reoxygenation control, n=4.
Figure 7. Cardiomyocyte contraction frequency. * - statistically significant difference compared to control normoxia when p<0.05, # - statistically significant difference compared to control hypoxialreoxygenation when p<0.05.
Figure 8. Cardiomyocyte peak times. * - statistically significant difference compared to control normoxia when p<0.05, # - statistically significant difference compared to control hypoxialreoxygenation when p<0.05.
Figure 9. Cardiomyocyte systolic times. * - statistically significant difference compared to control normoxia when p<0.05, # - statistically significant difference compared to control hypoxialreoxygenation when p<0.05.
Figure 10. Cardiomyocyte diastolic times. * - statistically significant difference compared to control normoxia when p<0.05, # - statistically significant difference compared to control hypoxialreoxygenation when p<0.05.
Figure 11. Mitochondrial basal respiration rate expressed in oxygen consumption rate (OCR). A - without adrenaline, B with adrenaline. * - indicates a statistical difference compared to the normoxia group when p < 0.05, # - indicates a statistical difference compared to the reoxygenation group when p < 0.05, n=3.
Figure 12. The rate of extracellular acidification (ECAR) corresponding to glycolytic activity of the cells. Basal respiration - before oligomycin addition and stressed respiration - after oligomycin addition. * - indicates a statistically significant difference compared to the normoxia group at p < 0.05, # - indicates a statistically significant difference compared to the hypoxialreoxygenation group at p < 0.05, n=3.
Figure 13. Glycolytic activity expressed as extracellular acidification rate (ECAR) measured simultaneously with the rate of mitochondrial respiration in cells with adrenaline. Basal respiration - before oligomycin addition and stress respiration after oligomycin addition. * - indicates a statistically significant difference compared to the normoxia group at p < 0.05, # - indicates a statistically significant difference compared to the hypoxialreoxygenation group at p < 0.05, n=3.

### DETAILED DESCRIPTION OF THE INVENTION

In the present disclosure, we extracted nanovesicles from dried *Melissa officinalis* plant and tested them on cultured primary cardiomyocytes subjected to I/R-mimicking conditions. We found that the nanovesicles preserve cardiomyocyte viability and contractility by protecting mitochondrial function during I/R injury. Melissa officinalis-derived nanovesicles serve as natural liposomes, protecting secondary metabolites and other biomolecules from environmental damage and promoting better transportability and availability of their bioactive content to cardiac cells.

### Definitions

Extracellular vesicles (EV), as used herein, are lipid bilayer particles that are naturally released from almost all types of cells but, unlike a cell, cannot replicate. EVs range in diameter from near the size of the smallest physically possible unilamellar liposome (around 20-30 nanometers) to as large as 10 microns or more. EVs with a diameter below 1 µm, may be referred to as nanovesicles or nanoparticles. The term "nanovesicle" is applied in the case of the inner volume of the particles is confirmed by transmission electron microscopy. If the samples are not examined this way, it is recommended to call them "nanoparticles". Extracellular particles below 20 nm in diameter are mostly nanoparticles without the inner volume.

Exosomes, as used herein, are EVs that are produced in the endosomal compartment of most eukaryotic cells. The diameter of exosomes is typically between 50 and 150 nm. Plant-derived nanovesicles, as used herein, are nanovesicles extracted from plant material regardless of intra- or extracellular origin.

Ischaemia-reperfusion injury (I/R), as used herein, is defined as the paradoxical exacerbation of cellular dysfunction and death, following restoration of oxygen to previously ischaemic tissues.

MO, as used herein, refers to *Melissa officinalis* plant, including any plant parts, such as aerial parts (for example, leaves, seeds, stems, etc) or underground parts (for example, roots). MON, as used herein, refer to *Melissa officinalis-*derived nanovesicles.

The present invention disclosure relates nanovesicles isolated from *Melissa officinalis* plant, compositions comprising said nanovesicles and uses thereof. In particular, the nanovesicles isolated from *Melissa officinalis* plant, as provided herein, were found to have advantageous effects on cells and tissue; they were able to enhance cell viability and regulate cell (for example, cardiomyocyte cell) contractility, thus protecting cells and tissues from damage or under stress conditions.

Specifically, the present invention disclosure provides nanovesicles obtained from *Melissa officinalis* plant. The nanovesicles when isolated from *Melissa officinalis* plant, are in the form of vesicles with lipid bilayer membrane and have a diameter of 10 to 700 nm. Preferably, the diameter of the nanovesicles of the present invention is from about 100 nm to about 350 nm. The nanovesicles may contain proteins in range of 1 µg to 1 mg of protein/1*10¹² particles and/or RNA in the range of 1 µg to 1 mg of nucleic acid/1*10¹² particles. The vesicles of the present invention can be obtained by using plant nanovesicle isolation methods available in the art or by other nanovesicle isolation methods yielding similar results, like ultracentrifugation, dialysis, tangential filtration, size exclusion chromatography, etc. The nanovesicles of the present invention can be obtained from various parts of *Melissa officinalis* plant, including aerial parts, such as leaves, seeds, stems, or underground parts, such as roots. Preferably, the nanovesicles from *Melissa officinalis* are obtained from aerial parts of the plant; more preferably they are obtained from the leaves of *Melissa officinalis.* The plant parts before isolation may be taken fresh or dried. Preferably, the nanovesicles of the present invention are isolated from dried plant parts, more preferably from dried aerial plant parts.

Another aspect of the present invention concerns the use of the nanovesicles from *Melissa officinalis* plant. The invention disclosure provides the nanovesicles from *Melissa officinalis* plant for use in therapy and/or medical treatment. In some embodiments, the nanovesicles are for use in prevention and/or treatment of ischaemia/reperfusion damage of cells or tissue. In some embodiments, the cells or tissue are from a muscle. In further embodiments, the muscle is cardiac muscle. In yet further embodiments, the nanovesicles for use in prevention and/or treatment of ischaemia/reperfusion damage of cells or tissue, wherein the tissue is an animal tissue, preferably wherein the tissue is a mammalian tissue, more preferably wherein the tissue is a human tissue; or wherein the cells are animal cells, preferably wherein the cells are mammalian cells, more preferably wherein the cells are human cells. In some embodiments, the nanovesicles are used in the concentrations from about 1.5*10¹⁰ to about 4.5*10¹⁰ nanovesicles/ml; for example, the nanovesicles are used at concentration of 1.5*10¹⁰, 2*10¹⁰, 2.5*10¹⁰, 3*10¹⁰, 3.5*10¹⁰, 4*10¹⁰ or 4.5*10¹⁰ nanovesicles/ml. Preferably, the nanovesicles are used at concentration of 3*10¹⁰ nanovesicles/ml.

In some embodiments, the nanovesicles are for use in prevention and/or treatment of ischaemia/reperfusion damage of cells or tissue, wherein the ischaemia/reperfusion damage is caused by myocardial infarction. In such cases the nanovesicles of the present invention can be used, for example, once blood flow to the cardiomyocytes has been restored. In such cases, the ischaemia/reperfusion damage is prevented or significantly attenuated by the presence of nanovesicles from *Melissa officinalis.*

In some embodiments, the nanovesicles are used in prevention and/or treatment of ischaemia/reperfusion damage of cells or tissue, wherein the ischaemia/reperfusion damage is caused by artificially induced hypoxic conditions. Artificially induced hypoxic conditions can occur, for example, in cases of surgery, when the blood flow is prevented to the organ of intervention. For example, during cardiac surgery, the heart is arrested; thus, artificially induced hypoxic conditions are created. For example, cardiac arrest during the cardiac surgery is performed by administration of cardioplegia solution. In some embodiments, the nanovesicles are used in prevention and/or treatment of ischaemia/reperfusion damage of cells or tissue, wherein the prevention and/or treatment of ischaemia/reperfusion damage is performed before, during or after surgery. In some embodiments, surgery is cardiac surgery.

In some embodiments, the nanovesicles from *Melissa officinalis* may be used to prevent deterioration of heart tissue. In some embodiments, the nanovesicles may be used to prevent deterioration of heart functions. In yet other embodiments, the nanovesicles may be used to prevent complications arising from deterioration of heart condition. In further embodiments, the nanovesicles may be used to treat heart arrhythmia. Specifically, in such cases, the prevention or attenuation of ischaemia/reperfusion damage by the nanovesicles from *Melissa officinalis* of the present invention may lead to the decreased probability of arrhythmia, as after treatment with the nanovesicles from *Melissa officinalis,* cardiomyocytes may contract less frequently.

The present invention disclosure further provides a composition for use in prevention and/or treatment of ischaemia/reperfusion damage of cells or tissue, characterised in that the composition contains nanovesicles obtained from *Melissa officinalis.* In some embodiments, the composition further comprises one or more additional active ingredients, provided that said one or more additional active ingredients are not encapsulated in said nanovesicles.

In some embodiments, the composition for use in prevention and/or treatment of ischaemia/reperfusion damage of cells or tissue comprises nanovesicles obtained from *Melissa officinalis* and is a cardioplegic composition. In some embodiments, such cardioplegic composition may contain components that are used in cardioplegic compositions. For example, the composition may contain potassium, magnesium, calcium, sodium or other salts used as electrolytes, mannitol, trehalose or similar sugars, lidocaine or any other anaesthetics performing similar functions.

In some embodiments, a pharmaceutical composition containing the nanovesicles obtained from *Melissa officinalis* and a pharmaceutically acceptable excipient is provided.

In a further aspect of the present invention, the use of nanovesicles obtained from *Melissa officinalis* to enhance cardiomyocyte viability and/or regulate cardiomyocyte contractility is disclosed. As such, for example, nanovesicles obtained from *Melissa officinalis* may regulate cardiomyocyte contractility homeostasis, keeping cell functions stable in presence of suppressing or stimulating factors. In some embodiments, the nanovesicles are used in the concentrations from about 1.5*10¹⁰ to about 4.5*10¹⁰ nanovesicles/ml; for example, the nanovesicles are used at concentration of 1.5*10¹⁰, 2*10¹⁰, 2.5*10¹⁰, 3*10¹⁰, 3.5*10¹⁰, 4*10¹⁰ or 4.5*10¹⁰ nanovesicles/ml. Preferably, the nanovesicles are used at concentration of 3*10¹⁰ nanovesicles/ml.

### EXAMPLES

### Methods

### Extraction of nanovesicles from Melissa Officinalis

Dried (at a temperature no higher than 40 °C) aerial parts of the *Melissa officinalis* (Germany, Alfred Galke GmbH) were used for the isolation of MON. Isolation was performed according to the manufa'turer's protocol using the ExoPLANT Lo Nanovesicle Isolation Kit (UAB Exolitus), briefly: the raw material was crushed in a mortar in an atmosphere of liquid nitrogen, mixed with stabilising reagent B and reagent A. The suspension was then mixed for 1 hour using a shaker at 10 rpm per min speed. Next, the sample was centrifuged for 1 hour at 3000 × *g* to remove undissolved plant parts quickly, and suspension was filtered through a 0.22 µm pore size syringe filter and mixed with with reagent C in a 1:1 ratio. The resulting solution was incubated for 16 hours at 4-8 °C. After the incubation sample was centrifuged at 3000 g for 1 hour, the supernatant was removed and the obtained nanovesicle pellet was resuspended in PBS.

### MON protein determination

Bradford method was used for protein quantification. Briefly: MON were mixed with 0.1% Triton solution in a ratio of 9:1 and incubated for 15-30 min at room temperature. After incubation, the solution was mixed with Bradford reagent in 9:1 ratio, and the absorbance was measured at a wavelength of 595 nm using PBS mixed with Bradford reagent as control. The concentration is calculated according to the equation of the calibration curve which is created by measuring known concentrations of bovine serum albumin.

### Evaluation of the size and number of particles in MON samples using the nanotracking analysis method (NTA)

Nanotracking analysis was applied to determine the size and concentration of nanovesicles using Nanosight NS300 device that counts the number of particles and estimates their size in the flowing liquid stream. The capillary of the device and the measuring cell were filled with PBS before measuring the sample. The MON suspension was diluted until between 100 and 10 particles at a time on the screen.

### Evaluation of the size distribution of nanoparticles in MON preparation by the dynamic light scattering method

Prior to measurement, the nanovesicle preparation is resuspended with a 27G insulin syringe for a few minutes to avoid clumping of vesicles. The nanovesicle preparation is diluted 20-50 times with PBS and measured using a disposable cuvette.

### Determination of particle morphology of Melissa officinalis preparation (Cryo-TEM)

Samples of MO nanoparticles were imaged by transmission electron microscopy. Isolated particles were homogenised for 5 min with 30G needle and mixed with 4% paraformaldehyde at the ratio of 1:1. This solution was applied on carbon-coated Formvar copper meshes FCFT200-Cu-50 200 MESH (Sigma-Aldrich, Taufkirchen Germany). The meshes were fixed in 1.7% glutaraldehyde solution for 5 min, washed twice in deionised water for 2 min, and stained with 2% uranyl acetate for 2 min. After staining, the meshes were incubated with freshly prepared 2.25% methylcellulose and 2% uranyl acetate in a v/v ratio of 4:1 for 10 min on an ice table. Prepared meshes were carefully dried on filter paper for 10 to 15 min and visualised using a transmission electron microscope Tecnai BioTwin Spirit G2 (FEI, Eindhoven, The Netherlands) on 80 kV voltage. Electron microscope images were taken with a bottom-mounted 16 MP TEM CCD camera Eagle 4K employing TIA (FEI, Eindhoven, The Netherlands).

### Evaluation of nucleic acid content of nanovesicles

TRIzol reagent was used for total nucleic acid determination following the manufacturer's protocol. Briefly: 100 µl of nanovesicle suspension is mixed with 900 µl of TRIzol reagent, incubated for 5 min, 200 µl of chloroform added, mixed, incubated for 2-3 min and centrifuged at 12,000 *× g* at 4 °C for 15 min. After centrifugation, the upper separated clear phase is transferred to another tube and mixed with 500 µl of isopropanol. The suspension is incubated for 10 min at 4 °C and centrifuged for 10 min 12,000 g at 4 °C. The obtained sediments are resuspended in 1 ml of 75% ethanol, vortexed for 5 min and centrifuged again for 5 min at 7500 g at 4 °C. The resulting sediments are dried in air and resuspended in 50 µl of ultrapure water, heating to 70 °C for 10 min. The light absorbance of the resulting suspension is measured at 260 nm and 280 nm using a Varioscan LUX plate reader and a µDrop^{™} plate. The amount of nucleic acids was calculated according to the formula A260x40= _ µg of nucleic acids/ml. The purity of the sample was estimated by the ratio of light absorption - 260 nm/280 nm. If the resulting ratio was between 1.8 and 2, the sample is considered clean for concentration assessment, and a ratio equal to 2 indicates a pure suspension of nucleic acids.

### Analysis of the chemical composition of nanovesicles

A Waters Acquity H-class UPLC System with a Waters Acquity Photodiode Array (PDA) detector was used for analysis of MON composition. Column used for analysis: Waters XTerra MS C18 4.6x100 mm, 3.5 µm sorbent particle size. MON was analysed for 4 active substances: caffeic acid, rosmarinic acid, p-coumaric acid and chlorogenic acid. Extraction of the active substances was performed from the lyophilised nanovesicle preparation by reconstitution in methanol and ultrasonication for 15 min.

### Preparation and culturing of primary culture of cardiomyocytes

Cardiomyocytes were isolated from 4-5 days old Wistar rats. All investigation procedures were performed according to the Republic of Lithuania Law on the Care, Keeping, and Use of Animals. The rodents were maintained at the Lithuanian University of Health Sciences animal house in agreement with the ARRIVE guidelines. The animal study protocol was approved by the Institutional Review Board of State Food and Veterinary Service, permission no. GS-195, date of approval 26 January 2022. 4-5 days old Wistar rat pups were used to prepare the primary culture of cardiomyocytes. Rat hearts were removed aseptically (in a laminar flow cabinet) and placed in chilled PBS. The hearts were cut vertically with a scalpel, and the tissues were rinsed in PBS to remove blood. The hearts were minced with surgical scissors, transferred to a centrifuge tube and centrifuged for 5 min at 500 × *g* speed. The pellet was resuspended with a mixture of trypsin-EDTA (2.5%) and collagenase (0.6 mg/ml). The hearts are left with the enzyme solution at +4 °C for 24 hours. After incubation, the tissue was triturated to a single cell suspension using 3 sizes of glass Pasteur pipettes with decreasing apertures and the resulting cell suspension was centrifuged for 5 min at 500 × *g* speed. The obtained pellet was resuspended in growth medium (high glucose DMEM + Glutamax supplemented with 10% FBS, 100 activity units/ml penicillin and 100 µg/ml streptomycin). Resuspended cells were sieved through a 40 µm pore size sieve to remove non-disrupted tissue pieces, leaving a single-cell suspension. A hemocytometer was used to estimate the cell concentration by diluting the cell suspension with 4% trypan blue dye in a ratio of 1:1. Live cells were counted using a light microscope, and cells were seeded for further experiments at a density of 250,000 cells/cm². The cells were grown for 7 days before the start of the experiments in an incubator in a 5% CO2 atmosphere at 37 °C, with the complete growth medium being changed every other day. Within 7 days, the culture was cleared of damaged cells, and the cells acquired characteristics of cardiomyocytes: striation and spontaneous cell contraction.

### Hypoxic conditions

Ischemia was simulated by maintaining cardiomyocytes in hypoxic conditions. 24 hours before cell incubation in hypoxic conditions, fresh growth medium was prepared and transferred to a hypoxia chamber (InvivO2, Baker Co), where an atmosphere of reduced oxygen (2% O2, 5% CO2, 93% N2 ) was maintained to equalise the dissolved oxygen content of the medium to that in the hypoxia chamber. The medium of experimental cardiomyocytes was replaced with this hypoxia-conditioned medium, and the cells were incubated for 24 hours in a hypoxia chamber.

### Fluorescent determination of metabolic activity

Metabolic activity of the cells was evaluated by a fluorescent PrestoBlue (Thermofisher Scientific) assay. To assess metabolic activity, 80,000 cells were seeded in 96-well flat-bottom plates. On the day of metabolic activity assessment, the medium was removed from the cells and a fresh medium with PrestoBlue dye mixture in a ratio of 9:1 was added and incubated for 30 min. Fluorescence intensity was measured at an excitation wavelength of 560 nm and an emission wavelength of 590 nm.

### Assessment of cardiomyocyte contractility

To evaluate changes in cell contractility cells were seeded on a gelatin-coated glass 4 well chamber slides (Nunc lab tek, Thermofisher scientific) and grown as described above. Before assessing cardiomyocyte contractions, the medium was changed to fresh medium containing 17 µg/ml adrenaline, which was incubated for 10 min at 37 °C. 30 s videos of cardiomyocytes visualised by Leica DMi1 (Leica, Germany) microscope were filmed with the Leica video system. Subsequently, the videos were evaluated with the ImageJ program Myocyter 1.3 add-on. All contracing cells or their clusters were manually circled, collecting a total of 30 such sites.

### Assessment of mitochondrial and glycolytic activity

The Seahorse XFp (Agilent Technologies Inc., USA) analyser and XFp Cell Mito Stress assay were used to assess the mitochondrial respiratory capacity. 1 hour prior to measurement, cell growth medium was changed to Seahorse XF DMEM supplemented with 10 mM glucose, 1 mM sodium pyruvate, 2 mM L-glutamine. The cells were incubated in a 37 °C incubator without CO₂ in order to remove carbon dioxide from the cells, which would affect measurements of pH changes by the analyser. At the start of the experiment, the sensor plate, hydrated overnight with distilled water, was prepared for calibration by adding Seahorse XF Calibrant to each well. The sensor prepared in this way is left for an hour in a 37 °C incubator without CO2. After an hour, automatic calibration of the device was performed, after which the calibration plate was replaced with an experimental plate, and the Mito Stress test was started. The data were analysed using Wawe software. Normalisation of the results was done by determining the total amount of protein in each of the tested wells of the plate according to the Bradford method.

### Immunocytochemical staining of cardiomyocytes

The quality of isolated primary culture of cardiomyocytes was evaluated in 2 ways: visually according to the occurrence of cell contraction and according to the cytoskeleton structure of the cells. The cells were fixed with 4% paraformaldehyde solution (dissolved in PBS) and incubated at room temperature for 5 min. Next, the cells were washed 3 times with PBS and incubated for 5 min. with 0.1 % Triton x-100 solution to permeabilise the cell membrane. Cells were washed again 3 times with PBS and incubated at +4°C with PBS containing 5% bovine serum albumin. Next cells were incubated for 1 h. with phalloidin conjugated to a TRITC red fluorescent label (FAK100 kit, Merck), diluted 1:1000 with PBS. After incubation with the dye, the cells were washed 3 times with PBS and visualised with a fluorescence microscope Carl Zeiss Axio Observer Z1 (Carl Zeiss AG, Germany).

### Statistical data analysis

Statistical data analysis was performed using IBM SPPS Statistic 26 programs; the mean, standard deviations and differences between the studied groups were evaluated using the Student's T-test. Averages with standard deviations of at least three replicates for each experiment were evaluated. Data were considered statistically significant when p<0.05.

### Results

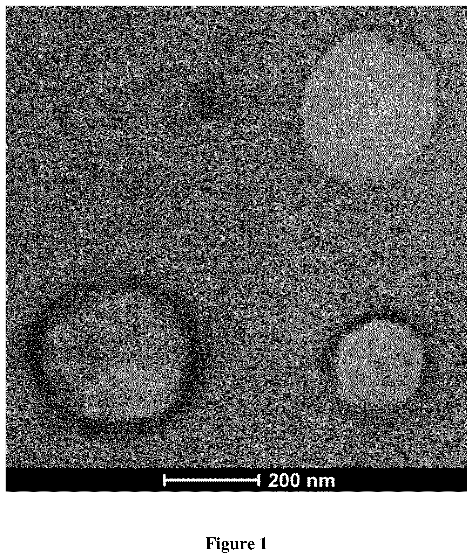

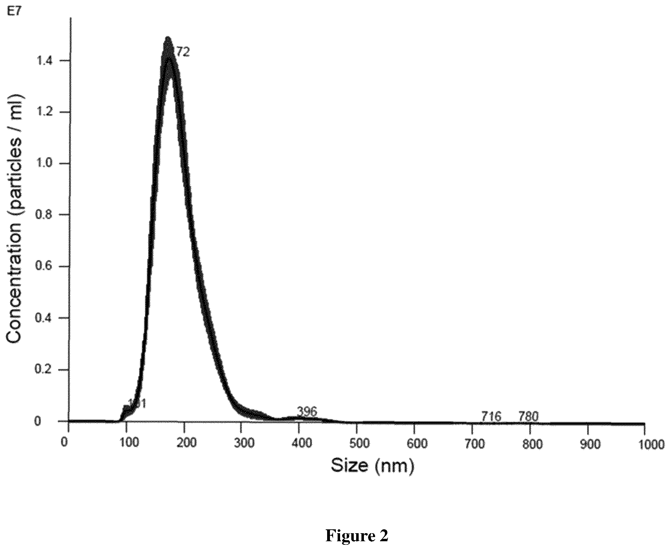

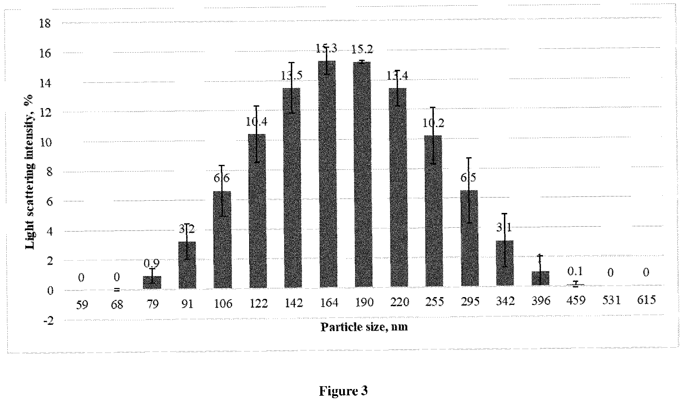

Vesicles containing a phospholipid bilayer with an average diameter ranging from 150 to 250 nm were identified using transmission electron microscopy (Figs. 1 and 2). NTA showed that 90% of the MON ranged in size between 146 and 244 nm, with a peak at 172 nm and an average size of about 191.3 nm for all particles (see Figure 2). Zetasizer analysis confirmed a similar particle size range (see Figure 3). The highest light scattering was found at 122 nm (10.4%), 142 nm (13.5%), 164 nm (15.3%), 190 nm (15.2%), 220 nm (13.4%) and 255 nm (10.2%). The peaks were at 164 and 190 nm. The obtained data show that the dominant vesicle size in the MON preparation was 122-255 nm.

MON numerical concentration was determined using NTA and averaged at 3.47 ×10¹² -+7.91 × 10¹² per 1 mg of total protein. MON contained 358,34±46,36 µg of total RNA per 1 mg of total protein, 248.8 ± 35 µg of Rosmarinic acid and 12.2 ± 6 µg of caffeic acid per 1 mg of total protein and contained traces of chlorogenic acid.

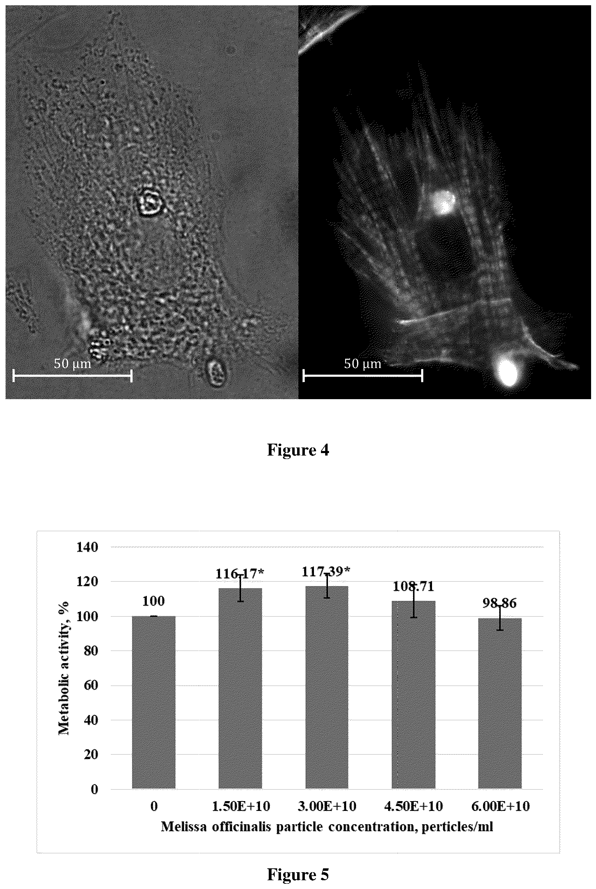

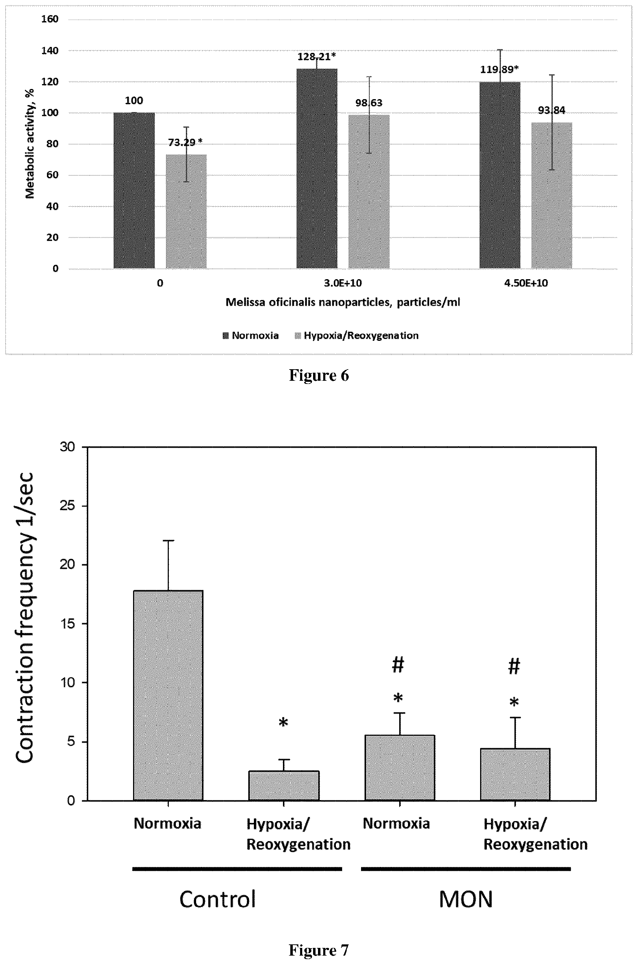

Immunostaining of primary cardiomyocyte culture showed cells with distinct actin patterns confirming mature cardiomyocytes after 7 days *in vitro* (Fig. 4). The maturity after day 7 *in vitro* was also confirmed by spontaneous cardymyocite beating. Several concentrations (1.5 * 10¹⁰, 3* 10¹⁰, 4.5* 10¹⁰, 6 * 10¹⁰ of MON particles/ml) were tested on primary cardiomyocyte cell culture to determine their effect on cell viability and metabolic activity using PrestoBlue cell viability reagent during normoxia and hypoxialreoxygenation conditions. Metabolic activity of cells in normoxia increased by 16.17±7.78% and 17.39±7.10%, respectively, in the presence of 1.5 * 10¹⁰, 3* 10¹⁰ of MON particles/ml concentration (Fig. 5). Hypoxia/reoxygenation significantly decreased the viability of cells compared to cells grown under normoxia conditions. Application of 3* 10¹⁰ particles/ml and 4.5* 10¹⁰ particles/ml concentrations of MON returned the viability of cells exposed to hypoxialreoxygenation to the level of control cells in normoxia (Fig. 6). In further experiments, 3* 10¹⁰ particles/ml concentration of MON was used.

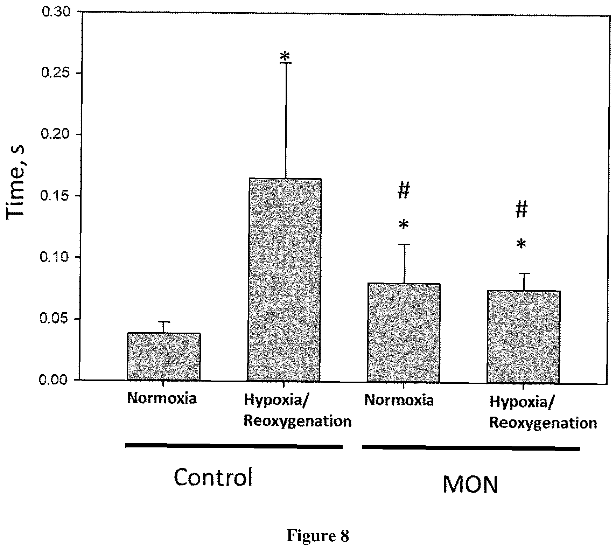

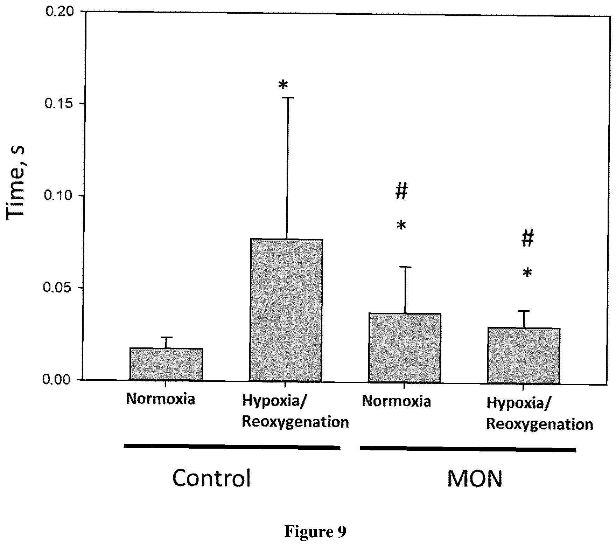

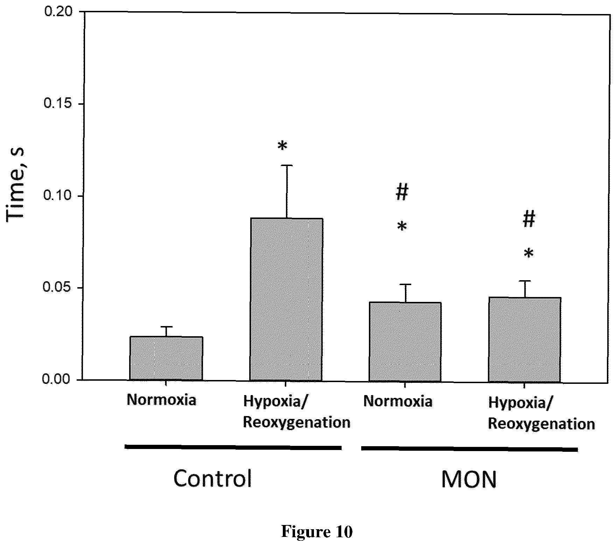

In order to evaluate cardiomyocyte contraction efficiency, their contraction frequency, peak, systole, and diastole times were measured. After hypoxialreoxygenation, cells stopped beating naturally; therefore, for contractility evaluation, adrenaline was used in all groups to stimulate cells. Hypoxia/reoxygenation decreased the contraction frequency several times from around 17.8 beats per second to only 2.5 times. MON prevented this hypoxia/reoxygenation effect by keeping the frequency in the hypoxialreoxygenation group up to 4.4 beats per second (Fig. 7). The data indicate MON could protect heart contractility during hypoxialreoxygenation. In contrast to the heartbeat-stimulating activity of hypoxia/reoxygenation-treated cells, MON lowered the normoxic adrenaline-stimulated cells' contraction frequency from 17.8 to 5.5 beats per second, making it close to a typical rat heartbeat rate of 5.3 beats per second (Fig. 7). Thus, MON protected cardiomyocyte activity loss caused by hypoxialreperfusion damage and prevented cell hyperactivity induced by overstimulation. Thus, MON can regulate cardiomyocyte contractility homeostasis, keeping cell functions stable in presence of suppressing or stimulating factors. Similar results could be seen in peak time, systolic time and diastolic time - all these times were prolonged by hypoxialreperfusion, but this was prevented by MON, bringing the times closer to that of the normoxic control group (Figs. 8 to 10).

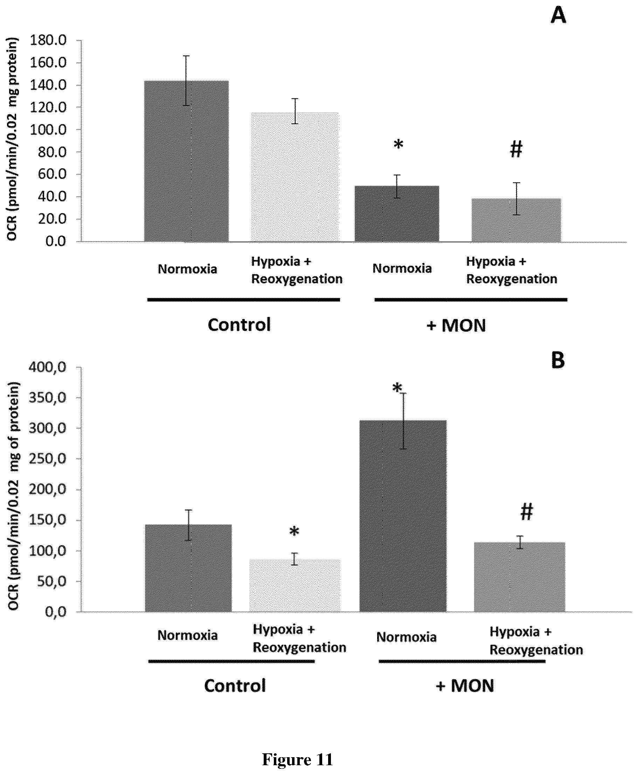

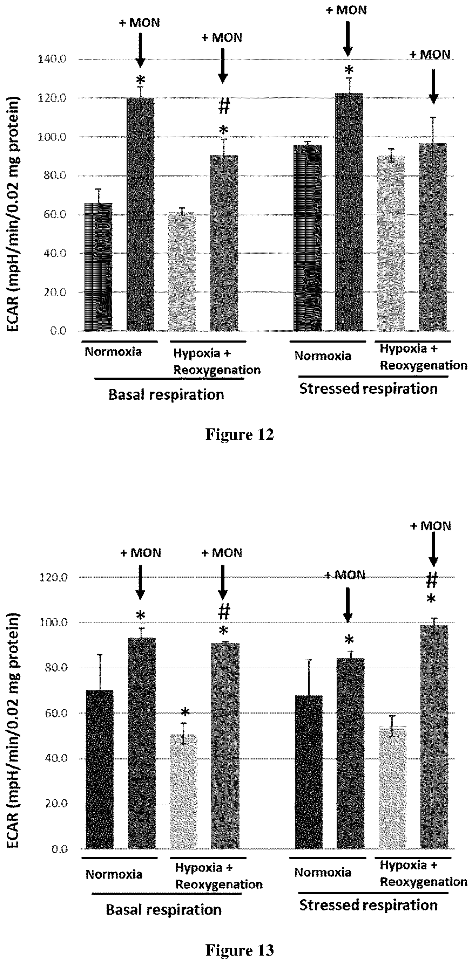

To assess how hypoxialreoxygenation changes mitochondrial activity and how this change is driven by MON, mitochondrial respiration in cell cultures was evaluated by a Seahorse analyser. Oxygen consumption rates (hereafter referred to as OCR in the graphs) were normalised to the total protein in the well of the assay plate. From the respiratory curves obtained, we can see that hypoxia with reoxygenation reduces the respiratory rate as expected. Still, the respiratory rate was even further reduced by MON, which strongly reduced basal respiratory rate (Fig. 11). Decreased cell and tissue activity requires less energy, which slows down cell processes. Reduced oxygen demand during ischemia would help cells protect themselves from oxidative damage when the blood flow is restored and reoxygenation starts. During normoxia, MON increased the rate of cellular acidification (abbreviated as ECAR, extracellular acidification rate in the graph) - this indicates that the rate of glycolysis, which does not require oxygen, increases in the cells. Increased pH was also seen in the MON-exposed group under hypoxic conditions during basal mitochondrial respiration (Figure 12). These results demonstrate that MON reduce mitochondrial respiration by enhancing glycolysis.

In order to assess whether the effect of MON on cardiomyocytes is irreversible or regulatory-protective, the experiments were repeated with the addition of adrenaline to stimulate cell contraction during measurements and to simulate heart load. Added adrenaline strongly increased the basal respiration of MON-treated cells in both normoxia and hypoxialreoxygenation. The level of mitochondrial respiration in MON-treated cells after hypoxialreoxygenation was restored to 80% of the control value. In cells kept under normoxic conditions, MON increased basal respiration mitochondrial 2-fold (Fig. 13). These changes with adrenaline indicate that MON-induced suppression of mitochondrial respiration is of a regulatory manner and can be restored when necessary. Moreover, the respiratory rates of the adrenaline-stimulated cells treated with MON are substantially higher than those without MON. Glycolysis in adrenaline-stimulated cells was decreased in the hypoxialreoxygenation group, but this decrease was prevented by MON.

The results obtained show that MON can protect the heart from damage caused by ischemial reperfusion injury by protecting cardiomyocyte viability, contractility, and mitochondrial function.

### References

1. Go AS, Mozaffarian D, Roger VL, Benjamin EJ, Berry JD et al; American Heart Association Statistics Committee and Stroke Statistics Subcommittee. Heart disease and stroke statistics--2014 update: a report from the American Heart Association. Circulation. 2014 Jan 21;129(3):e28-E292. doi: 10.1161/01.cir.0000441139.02102.80. Epub 2013 Dec 18. PMID: 24352519; PMCID: PMC5408159.Al-Salam S, Hashmi S: Myocardial Ischemia Reperfusion Injury: Apoptotic, Inflammatory and Oxidative Stress Role of Galectin-3. Cell Physiol Biochem 2018;50:1123-1139. doi: 10.1159/000494539
2. Jennings RB. Historical perspective on the pathology of myocardial ischemia/reperfusion injury. Circ Res. 2013 Aug 2;113(4):428-38. doi: 10.1161/CIRCRESAHA.113.300987. PMID: 23908330.
3. Shen D, He Z. Mesenchymal stem cell-derived exosomes regulate the polarisation and inflammatory response of macrophages via miR-21-5p to promote repair after myocardial reperfusion injury. Ann Transl Med. 2021 Aug;9(16):1323. doi: 10.21037/atm-21-3557. PMID: 34532460; PMCID: PMC8422151.
4. Kuznetsov AV, Javadov S, Margreiter R, Grimm M, Hagenbuchner J, Ausserlechner MJ. The Role of Mitochondria in the Mechanisms of Cardiac Ischemia-Reperfusion Injury. Antioxidants (Basel). 2019 Oct 6;8(10):454. doi: 10.3390/antiox8100454. PMID: 31590423; PMCID: PMC6826663.
5. Dad HA, Gu TW, Zhu AQ, Huang LQ, Peng LH. Plant Exosome-like Nanovesicles: Emerging Therapeutics and Drug Delivery Nanoplatforms. Mol Ther. 2021 Jan 6;29(1):13-31. doi: 10.1016/j.ymthe.2020.11.030. Epub 2020 Dec 3. PMID: 33278566; PMCID: PMC7791080.
6. Sedighi M, Faghihi M, Rafieian-Kopaei M, Rasoulian B, Nazari A. Cardioprotective Effect of Ethanolic Leaf Extract of Melissa Officinalis L Against Regional Ischemia-Induced Arrhythmia and Heart Injury after Five Days of Reperfusion in Rats. Iran J Pharm Res. 2019 Summer;18(3):1530-1542. doi: 10.22037/ijpr.2019.1100761. PMID: 32641961; PMCID: PMC6934948.

## Claims

1. Nanovesicles obtained from *Melissa officinalis* plant.

2. The nanovesicles according to claim 1 for use in therapy and/or medical treatment.

3. The nanovesicles for use according to claim 2, wherein therapy and/or medical treatment is prevention and/or treatment of ischaemia/reperfusion damage of cells or tissue.

4. The nanovesicles for use according to claim 3, wherein the cells or tissue are from a muscle.

5. The nanovesicles for use according to claim 4, wherein the muscle is cardiac muscle.

6. The nanovesicles for use according to any one of the preceding claims, wherein:
a. the tissue is an animal tissue, preferably wherein the tissue is a mammaliam tissue, more preferably wherein the tissue is a human tissue; or
b. the cells are animal cells, preferably wherein the cells are mammalian cells, more preferably wherein the cells are human cells.

7. The nanovesicles for use according to any one of preceding claims, wherein the ischaemia/reperfusion damage is caused by myocardial infarction.

8. The nanovesicles for use according to claim 4, wherein the ischaemia/reperfusion damage is caused by artificially induced hypoxic conditions.

9. The nanovesicles for use according to claim 3, wherein the prevention and/or treatment of ischaemia/reperfusion damage is performed before, during or after surgery.

10. The nanovesicles for use according to claim 9, wherein surgery is cardiac surgery.

11. A composition for use in prevention and/or treatment of ischaemia/reperfusion damage of cells or tissue, **characterised in that** the composition contains nanovesicles according to claim 1.

12. The composition for use according to claim 11, wherein the composition further comprises one or more additional active ingredients, provided that said one or more additional active ingredients are not encapsulated in said nanovesicles.

13. The composition for use according to claim 11 or 12, wherein the composition is a cardioplegic composition.

14. A pharmaceutical composition containing the nanovesicles according to claim 1 and a pharmaceutically acceptable excipient.

15. Use of nanovesicles according to claim 1 to enhance cardiomyocyte viability and/or regulate cardiomyocyte contractility.
